# EUROPEAN PATENT APPLICATION

(11) **EP 3 626 228 A1**
(43) Date of publication of application: **25.03.2020**
(21) Application number: 17910433.6
(22) Date of filing: 17.05.2017
(51) Int. Cl.: A61K 8/92, A61K 8/67, A61K 8/58, A61K 8/9783, A61Q 19/08

(54) **LIPOSOLUBLE ANTI-POLLUTION COSMETIC COMPOSITION, COSMETIC PRODUCT, USE AND METHOD FOR PREVENTING AND/OR TREATING SIGNS OF EXTRINSIC AGING**

(71) Applicant: Natura Cosméticos S.A., 05106-000 São Paulo SP (BR)
(72) Inventor: CAROLLO MONCAYO, Priscila, 05106-000 São Paulo - SP (BR); CARVALHÃES LAGO, Juliana, 05106-000 São Paulo - SP (BR); BAIONE DE MOURA, Soraya, 13208-703 Jundiaí - SP (BR)
(74) Representative: Simmons & Simmons
(86) International application number: PCT/BR2017/050118
(87) International publication number: WO 2018/209412

(57) **Abstract**

The present invention relates to lipid-soluble, anti-pollution cosmetic compositions as well as cosmetic products comprising the same, which are suitable for the treatment and prevention of signs of extrinsic aging, particularly caused by external aggressors from pollution.

## Description

### Field of the Invention

The present invention relates to lipid-soluble, anti-pollution cosmetic compositions as well as cosmetic products comprising the same, which are suitable for the treatment and prevention of signs of extrinsic aging, particularly caused by external aggressors from pollution.

### Background of the invention

The environment and our lifestyle affect our skin's health and quality. Throughout the day the skin is exposed to various types of agressors, particularly from pollution, which unbalances and accelerates aging.

Natural or chronological aging is the physiological decay process that takes place in our body over the years, also designated as intrinsic aging. The skin aging process induced by environmental factors is the so-called premature or extrinsic aging and can be distinguished from chronological or intrinsic aging by the characteristic signs of each type of aging. In extrinsic aging the signs are more pronounced on the skin, such as thicker and rougher skin, formation of dark spots and stains, telangiectasia (presence of tiny dilated blood vessels on the skin) in addition to sagging and deeper wrinkles.

Exposure to environmental factors, including air pollution, is responsible for skin damage leading to extrinsic aging, as listed below:
1. Free radical generation causes the activation of receptors on the skin surface that would be deactivated under normal conditions, causing the activation of factors that lead to a reduced synthesis of collagen, which is one of the main skin supporting proteins, as well as the oxidation of skin components that can lead to DNA damage.
2. Activation of aryl hydrocarbon receptor (AhR), which is a damage-causing receptor, can be triggered by the interaction between toxic components deposited on the surface of pollution particles and the skin, promoting a cascade of damage, such as synthesis of metalloproteinases responsible for the degradation of extracellular matrix proteins, including collagen and elastin, as well as stimulation of genes that promote skin pigmentation disorders and microdamage processes.
3. Decreased skin barrier quality due to the oxidation of important components such as squalene and vitamin E by pollution, as well as damage caused by the effects of UV radiation and air-conditioned environments (low air humidity).
4. Increased microdamage processes caused by cytosines that intensify processes of forming lines and wrinkles on the skin.

Although the body itself has defense systems intended to neutralize or remove environmental damage, detection of such damage under normal physiological conditions even in healthy organisms does not reach 100% efficiency, resulting in an imbalance between defenses and the resulting damage, which requires the use of complementary treatment systems.

Thus, there remains a need for cosmetic compositions to aid in the prevention and treatment of environmental damage that will invariably lead to extrinsic aging.

### Brief description of the figures

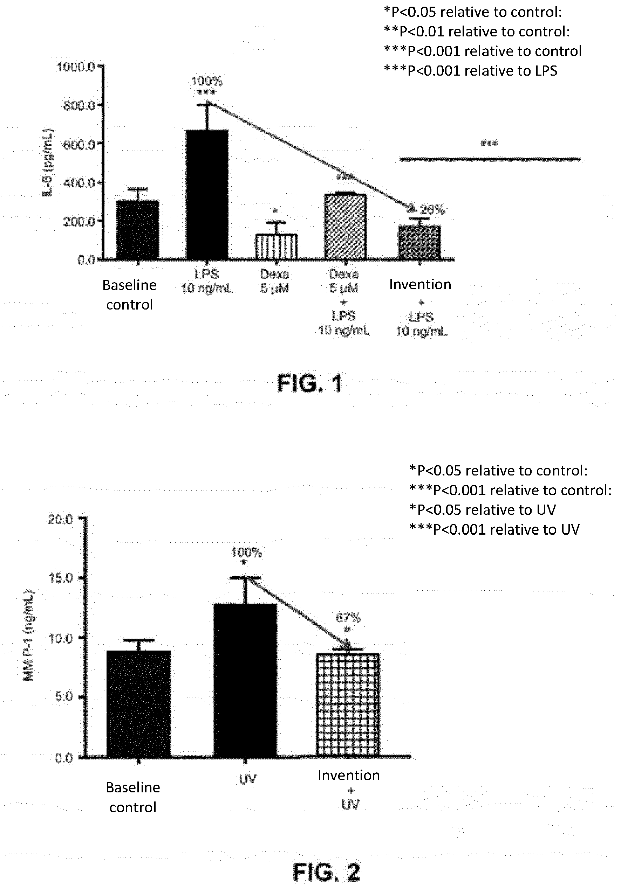

Figure 1 shows inhibition of IL-6 synthesis, a proinflammatory interleukin, characterizing an anti-inflammatory activity. The assay was performed using a facial oil containing 0.1% of the lipid-soluble anti-pollution cosmetic composition according to the present invention.

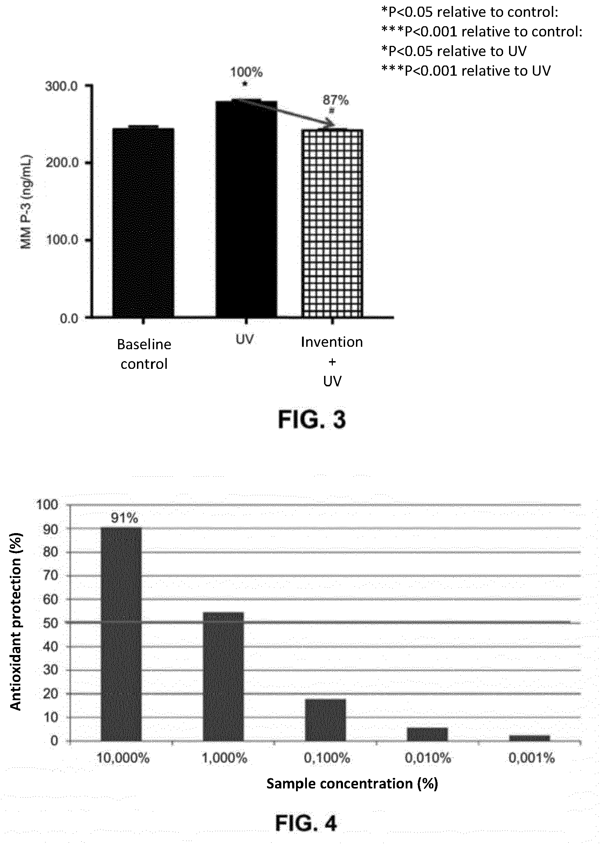

Figures 2 and 3 show MMP1 and MMP3 synthesis inhibition, respectively, which are metalloproteinases that characterize the protective activity of skin proteins. The assay was performed using a facial oil containing 0.1% of the liposoluble anti-pollution cosmetic composition according to the present invention.
Figure 4 shows a test using DPPH (2,2-diphenyl-1-picrylhydrazyl), which is based on the reduction of an ethanol-stable free radical by antioxidant substances. The assay was performed using a facial oil containing 0.1% of the liposoluble anti-pollution cosmetic composition according to the present invention.

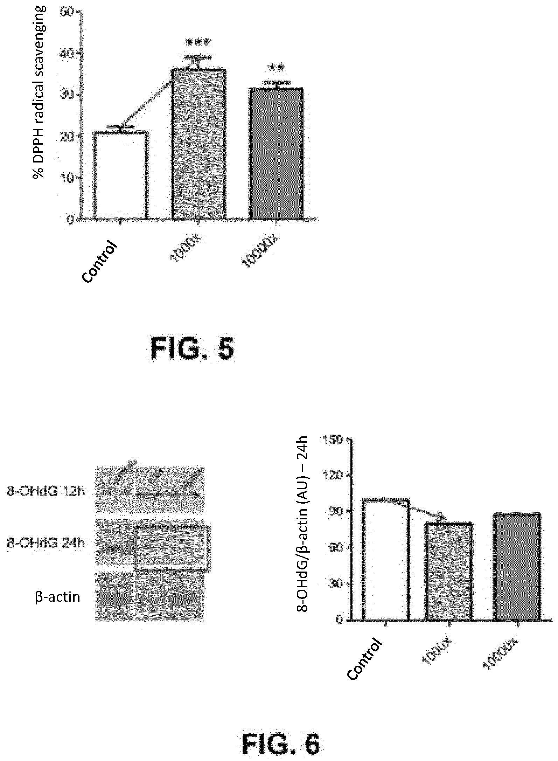

Figure 5 shows the total antioxidant ability by DPPH, representing a study method related to the total antioxidant defenses of the biological system. The assay was performed using a facial oil containing 0.1% of the lipid-soluble anti-pollution cosmetic composition according to the present invention.
Figure 6 shows the level of DNA protection by Western blotting for 8-OhdG, as this is the major metabolite generated by nucleic base oxidation. The assay was performed using a facial oil containing 0.1% of the lipid-soluble anti-pollution cosmetic composition according to the present invention.

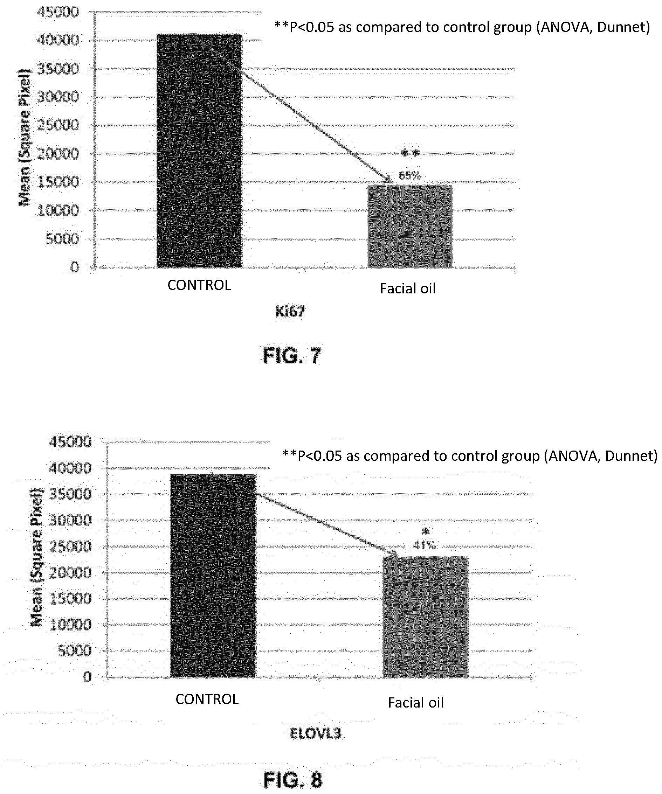

Figure 7 shows the Ki-67 antigen, a cell proliferation marker that is only present in dividing cells. Inhibition of Ki-67 synthesis indicates reduced proliferative index. The assay was performed using a facial oil containing 0.1% of the lipid-soluble anti-pollution cosmetic composition according to the present invention.
Figure 8 shows ELOVL3, which plays a role in the elongation of long chain fatty acids to provide precursors for the synthesis of sphingolipids and ceramides. The assay was performed using a facial oil containing 0.1% of the lipid-soluble anti-pollution cosmetic composition according to the present invention.

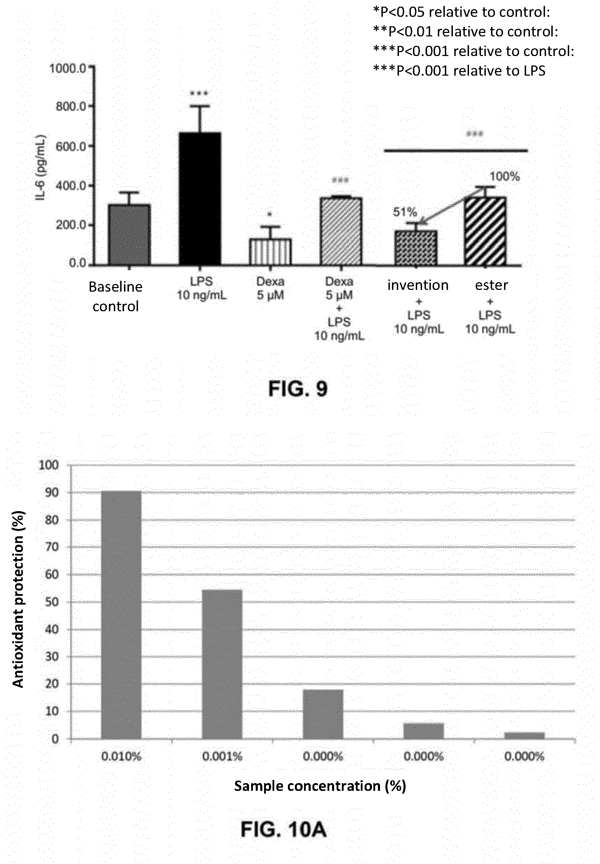

Figure 9 shows the inhibition IL6 synthesis, a proinflammatory interleukin that characterizes anti-inflammatory activity. The result has shown that testing with a facial oil containing 0.1% of the lipid-soluble anti-pollution cosmetic composition according to the present invention is more effective in reducing IL6 synthesis than hydroxycyclohexyl ethyltrisiloxane sapucainate (*Carpotroche brasiliensis* ester) alone.

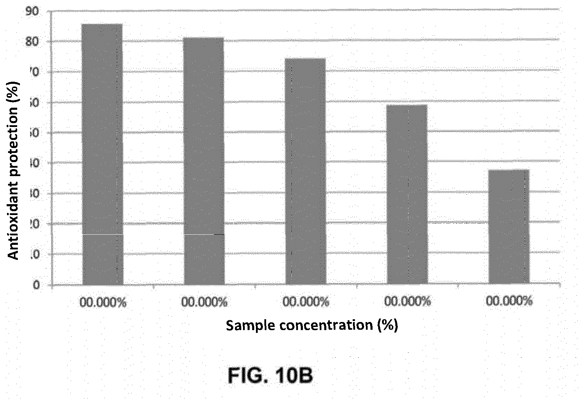

Figures 10A and 10B show a comparison of the antioxidant protection potential (%) between 0.2% ginger oil extract and the isolated polyphenol result.

### Description of the invention

Considering the main imbalances caused by pollution aggressors on the skin, which accelerate the extrinsic aging process, a lipid-soluble anti-pollution cosmetic composition has been developed in order to protect the skin from seven imbalances caused by pollution and its aggressors:
1. Protection of collagen fibers;
2. Protection of elastin fibers;
3. Protection against microdamage that intensify the formation of wrinkles;
4. Protection against oxidative stress;
5. Protection against damage to skin DNA;
6. Stimulation of the skin's natural antioxidant response; and
7. Prevention of pollution particles from interacting with the skin.

Thus, in a first aspect, the present invention relates to a lipid-soluble anti-pollution cosmetic composition, which may be made available through different cosmetic bases known in the art, whose anti-pollution effect is the result of a synergistic combination containing the following ingredients:
(a) at least one siliconized *Carpotroche brasiliensis* ester (sapucain), particularly hydroxycyclohexyl ethyltrisiloxane sapucainhate;
(b) at least one polyphenol, particularly from *Zingiber officinale* (ginger);
(c) at least one vitamin, particularly tocopheryl acetate; and
(d) at least one synthetic molecule.

The synthetic molecule according to the present invention is capable of stimulating CYP1A1, POMC, IL-6 and MMP-1 genes, being particularly selected from benzylidene dimethoxydimethylindanone.

The anti-pollution cosmetic composition according to the present invention is oil-soluble and may be incorporated into different cosmetic bases, for example, oily or emulsion bases, in an amount ranging from about 0.1 to about 10%, particularly about 2%.

In another aspect the present invention further relates to a cosmetic product comprising the anti-pollution cosmetic composition according to the present invention in a cosmetic, oily or emulsion base, in an amount ranging from about 0.1 to about 10%, particularly about 2%.

In another aspect, the present invention further relates to the use of the lipid-soluble anti-pollution cosmetic composition according to the present invention in the preparation of a cosmetic product useful in the treatment and prevention of signs of extrinsic aging, particularly caused by external pollution aggressors.

In another aspect, the present invention further relates to a method of preventing and/or treating the signs of extrinsic aging consisting of applying to the skin an effective amount of the lipid-soluble anti-pollution cosmetic composition according to the present invention.

The following examples, without limitation, illustrate the compositions according to the present invention as well as their effects, as described herein.

### EXAMPLES

### Example 1. In vitro test results of the anti-pollution technology

In order to investigate the biological protective potential of the lipid-soluble cosmetic composition according to the present invention, five major markers have been selected, which are known to cause skin damage when over-stimulated by exposure to polluted environments. Namely:

| Protein Markers | What does it do? | What is perceived on the skin? |
|---|---|---|
| Metalloproteinase 1 | Enzyme involved in extracellular matrix breakdown (collagen). | Wrinkles and sagging. Causes skin structural proteins to breakdown. |
| Metalloproteinase 3 | Enzyme involved in extracellular matrix breakdown (collagen, proteoglycans, laminin, fibronectin, gelatin and elastin). | Wrinkles and sagging. It causes skin structural proteins to breakdown. |
| Proinflammatory cytokine | Protein synthesized in areas of acute and chronic inflammation and is secreted into the extracellular environment (outside the cell). | It induces an inflammatory response, which can cause damage to dermal proteins. |
| It has antioxidant activity, stimulates endogenous antioxidants and protects the DNA. | It neutralizes the action of free radicals resulting from normal physiological processes. | It can cause protein degradation, breakdown of lipid cell walls and DNA damage. |

### Inhibition of proinflammatory cytokines and metalloproteinases

### Antiprotease (MMP1 and MMP3)

Metalloproteinase inhibition was determined from the synthesis of MMP1 and MMP3 in a primary culture of human dermal fibroblasts either exposed or not to ultraviolet radiation (UV) and commercially available immunoenzymatic (sandwich ELISA) kits were used (R&D Systems, Inc., Minneapolis, MN, USA; MMP-1 - Cod.: DY901 and MMP-3- Cod.: DY513) in the supernatant of fibroblast cultures treated with the products. MMP1 and MMP3 levels were calculated from the reference values obtained from a standard curve.

### Anti-Inflammatory (IL6)

The anti-inflammatory activity was determined from the synthesis of IL6 in a primary culture of human dermal fibroblasts either exposed or not to *Escherichia coli* lipopolysaccharide (LPS) and commercially available immunoenzymatic (sandwich ELISA) kits were used (R&D Systems, Inc., Minneapolis, MN, USA; IL-6 - Cod.: DY206-05) in the supernatant of fibroblast cultures treated with the products. IL6 levels were calculated from the reference values obtained in a standard curve.

Figure 1 shows inhibition of IL-6 synthesis, a proinflammatory interleukin, characterizing an anti-inflammatory activity. The assay was performed using a facial oil containing 0.1% of the lipid-soluble anti-pollution cosmetic composition according to the present invention.

Figures 2 and 3 show MMP1 and MMP3 synthesis inhibition, respectively, which are metalloproteinases that characterize the protective activity of skin proteins. The assay was performed using a facial oil containing 0.1% of the lipid-soluble anti-pollution cosmetic composition according to the present invention.

According to the presented results, the sample containing the lipid-soluble anti-pollution cosmetic composition according to the present invention in facial oil has shown excellent antiprotease and anti-inflammatory activity, reducing the increase in LPS-induced IL6 synthesis by 74%, UV-radiation-induced MMP1 synthesis by 33% and UV radiation-induced MMP3 synthesis by 13% as compared to the untreated damage control.

### Antioxidant activity

### DPPH

The test is based on the reduction reaction of free radical diphenyl picrylhydrazyl (DPPH) in an ethanolic medium with the raw materials or substances to be analyzed, the DPPH radical being reduced by these substances, thereby changing its shade and providing a measurement of the antioxidant protection. The result is expressed as % protection against the action of free radicals.

### DPPH in a cellular model

The total antioxidant capacity is assessed by the DPPH method. In this model, cell antioxidants directly react with the DPPH radical. Fibroblast line L929 was used in this study. The bioactive sample was directly solubilized in the culture medium and the control group received only culture medium and fetal bovine serum. Any increases observed as compared to the control group implies a gain in endogenous antioxidant capacity.

### Protection of genomic DNA in a cellular model

DNA protection was assessed by means of protein expression through the Western blotting method. The marker is visualized by the chemiluminescent reaction of peroxidase conjugated to the 8-OhdG marker antibody. A stronger band means greater DNA damage while a weaker band means less DNA damage.

Figure 4 shows a test using DPPH (2,2-diphenyl-1-picrylhydrazyl), which is based on the reduction of an ethanol-stable free radical by antioxidant substances.

Figure 5 shows the total antioxidant ability by DPPH, representing a study method related to the total antioxidant defenses of the biological system. The assay was performed using a facial oil containing 0.1% of the lipid-soluble anti-pollution cosmetic composition according to the present invention.

Figure 6 shows the level of DNA protection by Western blotting for 8-OhdG, as this is the major metabolite generated by nucleic base oxidation. The assay was performed using a facial oil containing 0.1% of the lipid-soluble anti-pollution cosmetic composition according to the present invention.

According to the results, the sample containing the lipid-soluble anti-pollution cosmetic composition in an anti-pollution facial oil product has shown excellent antioxidant activity in all protocols examined, resulting in 91% antioxidant protection against the action of free radicals, stimulating natural cellular antioxidants and protecting the genomic DNA against oxidative damage from external factors.

### Barrier strengthening protecting stimulus

Fatty acids act as structural partners of all cells and are extremely important in the cellular metabolism, especially those considered essential. Essential fatty acids can determine structural and functional changes in the phospholipid membrane, including immune system cells, modifying their stability, permeability, receptor and enzyme activity, transport with regulatory functions in addition to cellular metabolism. Seven Elovl (Elovl1-7) family enzymes found in mammals act as limiting enzymes for the elongation of C16, C18, or C20 carbon chains of saturated fatty acids. ELOVL3 acts to regulate adipogenesis progression, thereby stimulating adipogenesis and intracellular lipid accumulation, and plays a role in long chain fatty acid elongation to provide precursors for the synthesis of sphingolipids and ceram ides.

Ki-67 nuclear protein is expressed in all the active phases of the cell cycle (G1, S, G2 and mitosis), being absent from the resting phase only (G0). Its presence is closely associated with the cell cycle, being an important cell proliferation marker acting on the maintenance and/or regulation of the cell division cycle.

Figure 7 shows the Ki-67 antigen, a cell proliferation marker that is only present in dividing cells. Inhibition of synthesis of Ki-67 means a reduced proliferative index. The assay was performed using a facial oil containing 0.1% of the lipid-soluble anti-pollution cosmetic composition according to the present invention.

Figure 8 shows ELOVL3, which plays a role in the elongation of long chain fatty acids to provide precursors for the synthesis of sphingolipids and ceramides. The assay was performed using a facial oil containing 0.1% of the liposoluble anti-pollution cosmetic composition according to the present invention.

According to the results, the sample containing the lipid-soluble anti-pollution cosmetic composition according to the present invention in an anti-pollution facial oil product has shown Ki-67 and ELOVL3 protein synthesis inhibition in human skin fragments as compared to the control group.

*In vitro* tests have shown in general the lipid-soluble anti-pollution cosmetic composition according to the present invention has acted on the skin's main biological mechanisms that are affected by exposure to external aggressors such as pollution, acting on:
- reduction of IL6 synthesis by 74% as compared to cells exposed to damage (microdamage reduced by 74%);
- reduction of MMP1 synthesis by 33% as compared to cells exposed to damage (collagen-degrading enzymes reduced by 33%)
- reduction of MMP3 synthesis as compared to cells exposed to damage (it reduces enzymes that degrade dermal proteins (collagen, proteoglycans, fibronectin, laminin, and elastin);
- it has 91% antioxidant protection (protects free radical action by 91%);
- stimulation of the skin's natural antioxidants; - DNA protection against damage caused by external factors;
- reduction of the synthesis of Ki67 and ELOVL3 slowing down the cell proliferation metabolism.

### Biological anti-pollution potential of the lipid-soluble anti-pollution cosmetic composition according to the present invention versus isolated active ingredients

In this study comparative tests were performed between the lipid-soluble anti-pollution cosmetic composition according to the present invention and the active ingredients alone.

Figure 9 shows the inhibition IL6 synthesis, a proinflammatory interleukin that characterizes anti-inflammatory activity. The result has shown that testing with a facial oil containing 0.1% of the lipid-soluble anti-pollution cosmetic composition according to the present invention is more effective in reducing IL6 synthesis than hydroxycyclohexyl ethyltrisiloxane sapucainate (*Carpotroche brasiliensis* ester) alone.

Figures 10A and 10B show a comparison of the antioxidant protection potential (%) between 0.2% ginger oil extract and the isolated polyphenol result.

Higher potency of the lipid-soluble anti-pollution cosmetic composition according to the present invention was observed as compared to the active ingredients alone, siliconized sapucain ester and ginger polyphenols in all mechanisms evaluated herein. These actives are known to act on IL-6 inhibition mechanism and antioxidant action, respectively. The lipid-soluble anti-pollution cosmetic composition according to the present invention also exhibited a broader range of functionality on the skin's protecting mechanisms against pollution-caused damage as compared to the active ingredients alone.

From the teachings provided in the text and the examples presented herein the person skilled in the art will readily appreciate the advantages of the invention and will be able to propose variations and equivalent embodiment alternatives without departing from the scope of the invention as defined in the appended claims.

## Claims

1. A LIPID-SOLUBLE ANTI-POLLUTION COSMETIC COMPOSITION **characterized in that** it comprises:
(a) at least one siliconized *Carpotroche brasiliensis* ester;
(b) at least one polyphenol;
(c) at least one vitamin; and
(d) at least one synthetic molecule capable of stimulating CYP1A1, POMC, IL-6 and MMP-1 genes.

2. The composition of Claim 1, **characterized in that** it comprises:
(a) hydroxycyclohexyl ethyltrisiloxane sapucainate;
(b) at least one *Zingiber officinale* polyphenol;
(c) tocopheryl acetate; and
(d) benzylidene dimethoxydimethylindanone.

3. A COSMETIC PRODUCT **characterized in that** it comprises the lipid-soluble anti-pollution cosmetic composition of claim 1 or 2 and a cosmetically acceptable carrier.

4. The PRODUCT of claim 3, **characterized in that** it comprises from about 0.1 to about 10% of the cosmetic composition of claim 1 or claim 2.

5. The PRODUCT of claim 4, **characterized in that** it comprises about 2% of the cosmetic composition of claim 1 or 2.

6. USE of the cosmetic composition of claim 1 or 2, **characterized in that** it is in the manufacture of a cosmetic product useful in the treatment and prevention of signs of extrinsic aging.

7. A METHOD OF PREVENTING AND/OR TREATING SIGNS OF EXTRINSIC AGING **characterized in that** it consists of applying on the skin an effective amount of the cosmetic composition of claim 1 or 2.
